# EUROPEAN PATENT SPECIFICATION

(11) **EP 3 586 130 B1**
(45) Date of publication and mention of the grant of the patent: **04.11.2020**
(21) Application number: 18705740.1
(22) Date of filing: 05.02.2018
(51) Int. Cl.: G01N 33/49

(54) **BLOOD COAGULATION MEASUREMENT CARTRIDGE**
BLUTGERINNUNGSMESSKARTUSCHE
CARTOUCHE DE MESURE DE COAGULATION SANGUINE

(30) Priority: 21.02.2017 JP 2017029849
(43) Date of publication of application: 01.01.2020
(73) Proprietor: Sony Corporation, Tokyo 108-0075 (JP)
(72) Inventor: HIDAKA, Isao, Tokyo 108-0075 (JP); TERAKADO, Daisuke, Tokyo 108-0075 (JP)
(74) Representative: D Young & Co LLP
(86) International application number: PCT/JP2018/003772
(87) International publication number: WO 2018/155145

(56) References cited:
- EP-A1- 2 947 451
- JP-A- 2004 205 462
- JP-A- 2015 148 594
- US-A- 3 560 162

## Description

### [CROSS REFERENCE TO RELATED APPLICATIONS]

This application claims priority to Japanese Patent Application JP 2017-029849 filed on February 21, 2017.

### [Technical Field]

The present invention relates to an electrical measurement cartridge for electrical measurement. More particularly, the present invention relates to an electrical measurement cartridge having excellent sealing characteristics, and an electrical measurement device, an electrical measurement kit, and an electrical measurement method that use the electrical measurement cartridge.

### [Background Art]

These days, the electrical characteristics of a sample are measured, and the physical properties of a biological sample are determined from a result of the measurement, or the type of the cells or the like contained in a biological sample is determined from the result of the measurement (PTL 1, for example). In addition, the electrical characteristics to be measured may be complex permittivity and its frequency dispersion (a dielectric spectrum), for example. Complex permittivity and its frequency dispersion are normally calculated by measuring the complex capacitance or the complex impedance between electrodes, using a solution container or the like that has the electrodes for applying voltage to a solution.

As an example of a device that conducts such measurement, PTL 2 discloses "a blood coagulation system analyzing device that includes: a pair of electrodes; an application unit that applies alternating voltage to the pair of electrodes at predetermined time intervals; a measurement unit that measures the permittivity of blood placed between the pair of electrodes; and an analysis unit that analyzes the function of a blood coagulation system, using the permittivity of the blood measured at the predetermined time intervals after the anticoagulant acting on the blood is removed".

In a case where measurement is conducted with the above described device or the like, an electrical measurement container that contains a sample is normally used. For example, PTL 3 discloses "an electrical measurement cartridge that includes: a container having an opening portion and a sample holding portion; a sealing portion that seals at least part of the sample holding portion; and an electrode secured to the sample holding portion, in which the sealing portion separates the sealed portion of the sample holding portion and the electrode from each other".

In such an electrical measurement cartridge including a container and a lid unit, however, the top edge the lid unit is normally formed with an elastic packing, to improve sealing characteristics. In such a structure, the lid unit needs to be formed with two members of different materials. Therefore, there are additional necessary steps for two-color molding and assembling to be performed after the respective components are manufactured.

If the packing is eliminated, and the lid unit can be formed with one material to counter this problem, the manufacturing process can be simplified, and a higher economic efficiency can be expected. In such a case, however, it is difficult to achieve excellent sealing characteristics with an electrical measurement cartridge having a conventional structure, and there is a need to further improve the structure.

### [Citation List]

### [Patent Literature]

[PTL 1]
   JP 2009-042141 A
[PTL 2]
   JP 2010-181400 A
[PTL 3]
   JP 2015-148594 A

Prior art also includes JP 2004 205462A; EP 2947451A1; and US 3560162A.

### [Summary]

### [Technical Problem]

In view of this, the present invention aims to provide an electrical measurement cartridge with excellent sealing characteristics.

### [Solution to Problem]

Aspects of the present invention are defined by the appended Claims.

### [Advantageous Effects of Invention]

According to an embodiment of the present invention, an electrical measurement cartridge that has excellent sealing characteristics can be provided. It should be noted that effects of the present invention are not limited to the effect described above, and may include any of the effects described herein.

### Brief Description of Drawings

[fig.1]Fig. 1 is a perspective view of the exterior of an embodiment of an electrical measurement cartridge 1 according to an embodiment of the present invention

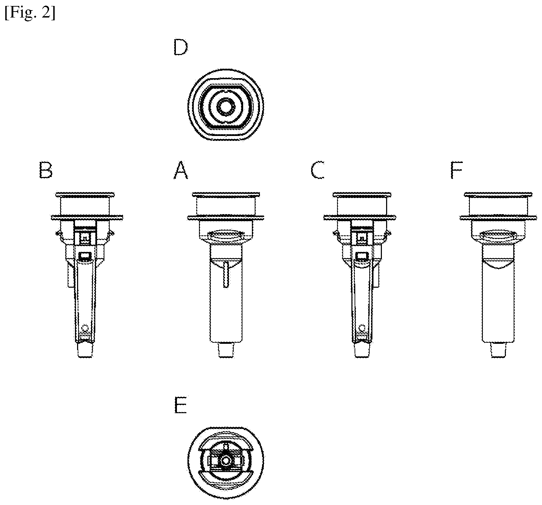

[fig.2]Figs. 2A to 2F are views of the embodiment shown in Fig. 1, seen from six different directions.
[fig.3]Fig. 3 is a vertical cross-sectional view taken along the center of the embodiment shown in Fig. 2B (left side view).
[fig.4]Fig. 4 is an enlarged view of the portion defined by A-A and B-B in Fig. 3.

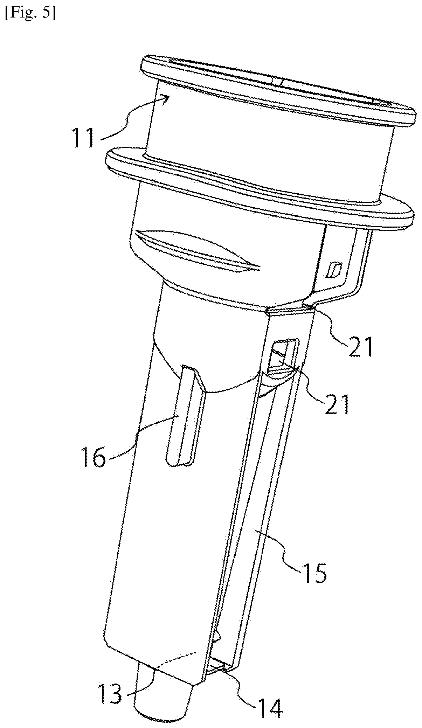

[fig.5]Fig. 5 is a perspective view of the exterior of the container 11 in the embodiment shown in Fig. 1.

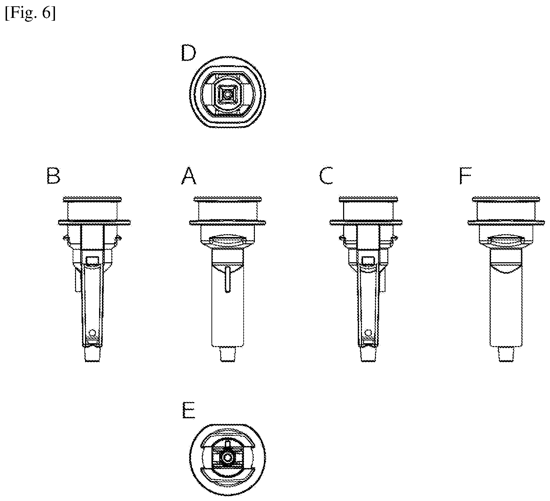

[fig.6]Figs. 6A to 6F are views of the container 11 shown in Fig. 5, seen from six different directions.
[fig.7]Fig. 7 is a diagram showing a modification of the container 11.
[fig.8]Fig. 8 is a perspective view of the exterior of the lid unit 12 in the embodiment shown in Fig. 1.
[fig.9]Figs. 9A to 9F are views of the lid unit 12 shown in Fig. 8, seen from six different directions.
[fig. 10] Fig. 10 is a diagram showing a modification of the sealing portion 121.
[fig. 11| Figs. 11A to 11C are diagrams showing modifications of the shaft portion 122.
[fig.12] Figs. 11A to 11C are diagrams showing modifications of the spring portion 123.
[fig.13]Fig. 13 is a schematic conceptual diagram schematically showing an embodiment of an electrical measurement device 10 according to an embodiment of the present invention.
[fig.14]Fig. 14 is a diagram showing an embodiment of an electrical measurement kit K according to an embodiment of the present invention.

### Description of Embodiments

The following is a description of preferred embodiments for carrying out the present invention, with reference to the accompanying drawings. It should be noted that the embodiments described below are typical examples of embodiments of the present invention, and do not narrow the interpretation of the scope of the present invention. Meanwhile, explanation will be made in the following order.
1. Electrical measurement cartridge 1
   (1) Container 11
      (1-1) Opening portion 111
      (1-2) Sample holding portion 112
   (2) Lid unit 12
      (2-1) Sealing portion 121
      (2-2) Shaft portion 122
      (2-3) Spring portion 123
   (3) Electrodes 13
   (4) Securing mechanism 2
      (4-1) Recess portions 21
      (4-2) Claws 22
   (5) Stress generation mechanism
   (6) Connecting portions 14
   (7) Protecting portions 15
   (8) Guiding portions 16
   (9) Sample
   (10) Reagent
2. Electrical measurement device 10
   (1) Cartridge insertion unit 3
   (2) Application unit 4
   (3) Measurement unit 5
   (4) Unsealing mechanism
   (5) Analysis unit 6
   (6) Others
3. Electrical measurement kit K
   (1) Sample introduction member 7
4. Electrical measurement method

### 1. Electrical measurement cartridge 1

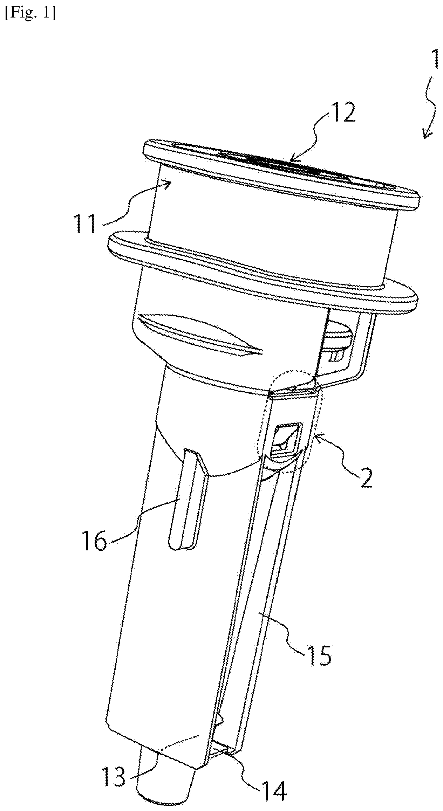

Fig. 1 is a perspective view of the exterior of an embodiment of an electrical measurement cartridge 1 according to an embodiment of the present invention The electrical measurement cartridge 1 according to an embodiment of the present invention is a cartridge that is used for holding a sample when the electrical characteristics of the sample are measured. The electrical measurement cartridge 1 according to an embodiment of the present invention includes at least a container 11, a lid unit 12, and electrodes 13, and has a securing mechanism and a stress generation mechanism. In addition, the electrical measurement cartridge 1 may further include connecting portions 14, protecting portions 15, and guiding portions 16, as necessary.

It should be noted that the sample and the reagent described layer are not included in the electrical measurement cartridge 1 according to an embodiment of the present invention. Also, the electrical measurement cartridge 1 according to an embodiment of the present invention can be regarded as an assembly formed with the container 11 and the lid unit 12.

In the description below, the respective components of the electrical measurement cartridge 1 according to an embodiment of the present invention will be explained in detail.

### (1) Container 11

The container 11 is a component that includes an opening portion 111 and a sample holding portion 112, and enables introduction and holding of a sample and/or a reagent.

Although materials that can be used as the container 11 according to an embodiment of the present invention are not limited to any particular materials, a resin may be used to form the container 11.

Resins that can be used as the container 11 are not limited to any particular types, and one or two or more types of resins that can be used for holding a sample can be freely selected. Examples of such resins include hydrophobic, insulating polymers, copolymers, and blended polymers, such as polypropylene, polymethylmethacrylate, polystyrene, acrylic, polysulfone, and polytetrafluoroethylene.

The container 11 is preferably formed with one or more resins selected from the group consisting of polypropylene, polystyrene, acrylic, and polysulfone. These resins have low coagulation activity with respect to blood, and accordingly, can be appropriately used for measurement in a case where a blood sample is selected as a sample.

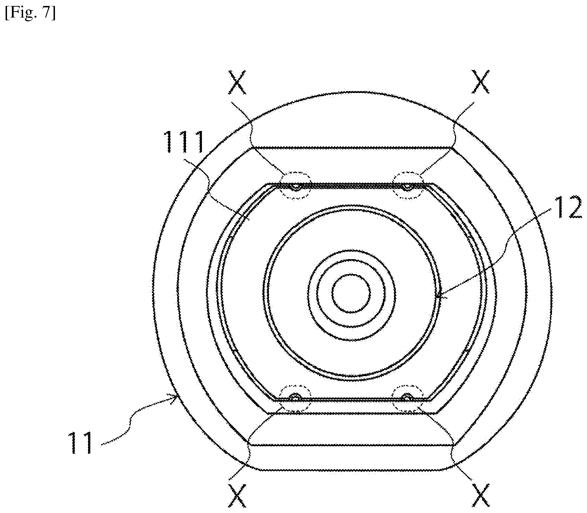

Substantially semicircular ribs (denoted by X in Fig. 7) may be formed at part of the container 11, as shown in Fig. 7. The ribs may be used as a positioning mechanism when the lid unit 12 is engaged. However, some or all of the ribs may be made to dig into the lid unit 12 so that the container 11 and the lid unit 12 can be more firmly engaged with each other. With this configuration, the lid unit 12 can be prevented from wobbling.

Also, instead of the above rib-like portions, some or all of the portions equivalent to X in Fig. 7 may be shaped into undercut portions, to add snap-fit portions. Further, the locations of the portions are not limited to those shown in the drawing, and snap-fit portions to be engaged with the lid unit 12 may be added to any portions on the inner sidewall of the container 11.

In the description below, the respective portions of the container 11 will be explained in detail.

### (1-1) Opening portion 111

The opening portion 111 is a portion through which the current sample or reagent to be measured can be introduced.

A method of introducing a sample or a reagent is not limited to any particular method, and a sample or a reagent can be introduced by any method suitable for the form of the container 11. For example, a sample or a reagent can be introduced with a pipette or the like.

### (1-2) Sample holding portion 112

The sample holding portion 112 is a portion by which the current sample or reagent to be measured can be held.

The sample holding portion 112 is not limited to any particular form, and can be freely designed in accordance with the type of the sample, the measurement method, the electrical measurement device being used, and the like. Examples of forms of the sample holding portion 112 include a cylindrical form, a polygonal cylindrical form having a polygonal (triangular, rectangular, or higher-order) cross-section, a conical form, a polygonal conical form having a polygonal (triangular, rectangular, or higher-order) cross-section, and a combination of one or two or more of these forms.

In the present invention, the form of the sample holding portion 112 is preferably such that at least the portion on which the later described electrodes 13 are disposed has a flat form. The reasons for this are described below in detail.

An electrode that is used in electrical measurement is normally in a flat form or a plate-like form. In a case where a cylindrical form is selected as the form of the sample holding portion 112, the electrodes 13 in a flat or plate-like form are attached to a curved portion, and therefore, the manufacturing process becomes very complicated. Furthermore, if the electrodes 13 in a flat or plate-like form are attached to a curved portion of the sample holding portion 112, there is a high possibility that a step will be formed at the connecting portions between the sample holding portion 112 and the electrodes 13, and the measurement accuracy in the electrical measurement might become lower in some cases. Therefore, in the sample holding portion 112, at least the portion on which the electrodes 13 are disposed is shaped into a flat form. Thus, the process of manufacturing the electrical measurement cartridge 1 can be simplified, and the measurement accuracy can be increased.

In the present invention, when electrical measurement of various kinds is conducted, a sample is held by the sample holding portion 112. Therefore, the sample holding portion 112 is preferably designed to be hermetically sealed while holding a sample. However, if the sample holding portion 112 can stay still during the time of measurement of various electrical characteristics of a sample, and does not affect the measurement, the sample holding portion 112 may not be able to be hermetically sealed.

### (2) Lid unit 12

The lid unit 12 includes at least a sealing portion 121 and a shaft portion 122. The lid unit 12 may further include a spring portion 123 and the like, as necessary.

In the lid unit 12 of the present technology, the sealing portion 121 and the other portions may be made of the same material. As described above, the top edge (the portion equivalent to the sealing portion 121 in the present technology) of a lid unit is normally formed with an elastic packing, to improve sealing characteristics. In such a structure, the lid unit needs to be formed with two members of different materials. Therefore, there are additional necessary steps for two-color molding and assembling to be performed after the respective components are manufactured. In the present invention, on the other hand, the lid unit 12 is formed with one material. Thus, the manufacturing process can be simplified, and a higher economic efficiency can be achieved.

Although materials that can be used as the lid unit 12 are not limited to any particular materials, a resin may be used to form the lid unit 12. Further, the container 11 and the lid unit 12 may be formed with one resin. Thus, the manufacturing process can be simplified, and a higher economic efficiency can be achieved. It should be noted that the types of resins and the preferred resins and the like are similar to those described above, and therefore, explanation of them is not repeated herein.

Further, in the electrical measurement cartridge 1 according to an embodiment of the present invention, a reagent can be sealed in the sealed portion of the sample holding portion 112, as necessary. In the present invention a method of sealing a reagent is not limited to any particular method, but it is preferable to seal a reagent beforehand at the time of manufacturing of the cartridge 1.

It should be noted that, when electrical measurement is conducted with the electrical measurement cartridge 1 according to an embodiment of the present invention the cartridge 1 holds a sample in the sample holding portion 112, and the lid unit 12 is removed. It should be noted that, in the electrical measurement cartridge 1 according to an embodiment of the present invention the lid unit 12 that is removed once can be reattached to the container 11 after electrical measurement.

In the description below, the respective portions of the lid unit 12 will be explained in detail.

### (2-1) Sealing portion 121

The sealing portion 121 is a portion that seals at least part of the sample holding portion 112.

The sealing portion 121 is not limited to any particular form as long as being capable of sealing part of the sample holding portion 112, and can be freely designed in accordance with the type of the sample, the measurement method, the electrical measurement device being used, and the like. Examples of forms of the sealing portion 121 include a cylindrical form, a polygonal cylindrical form having a polygonal (triangular, rectangular, or higher-order) cross-section, a conical form, a polygonal conical form having a polygonal (triangular, rectangular, or higher-order) cross-section, and a combination of one or two or more of these forms.

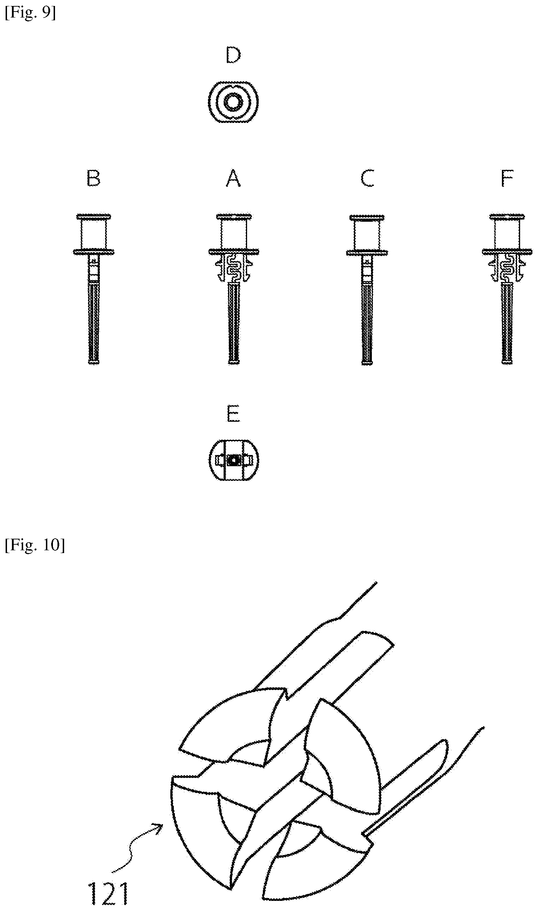

The sealing portion 121 may have a slotted form having large slits as shown in Fig. 10. Having this form, the sealing portion 121 is narrowed when a user pushes the lid unit 12 into the container 11. Thus, the lid unit 12 can be prevented from wobbling by virtue of the repulsive force.

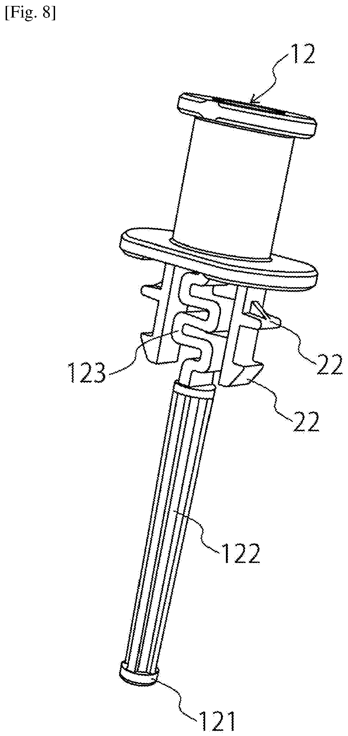

Further, the sealing portion 121 may be slightly larger than that of the embodiment shown in Fig. 8, and may be formed with a thin plate. Having this form, the sealing portion 121 is deformed when a user pushes the lid unit 12 into the container 11. Thus, the sealing characteristics may be improved, and a repulsive force may be generated. Further, the sealing portion 121 may be slightly larger than that of the embodiment shown in Fig. 8, and a hollow may be formed inside the sealing portion 121. Having this form, the sealing portion 121 is deformed when a user pushes the lid unit 12 into the container 11, as with the above described form. Thus, the sealing characteristics may be improved, and a repulsive force may be generated.

Although materials that can be used as the sealing portion 121 are not limited to any particular materials, a resin may be used to form the sealing portion 121. It should be noted that the types of resins and the preferred resins and the like are similar to those described above, and therefore, explanation of them is not repeated herein.

The container 11 has a slope at a portion that is part of the inner sidewall and is in contact with the sealing portion 121. The slope spreads from the container bottom toward the opening portion 111. The sealing portion 121 also has a slope at a portion that is part of the outer sidewall and is in contact with the container 11. The slope spreads from the bottom of the sealing portion toward the top portion of the sealing portion 121. The slope of the sealing portion 121 is preferably gentler than the slope of the container 11. With this arrangement, a space can be formed between the portion that is part of the inner sidewall of the container 11 and is in contact with the sealing portion 121, and the portion that is part of the outer sidewall of the sealing portion 121 and is in contact with the container 11. Therefore, even if a burr is formed at the time of formation of the sealing portion 121, the sealing characteristics of the sealing portion 121 are not affected. Thus, excellent sealing characteristics can be maintained, while the management of the manufacturing process is simplified, and a higher economic efficiency is achieved.

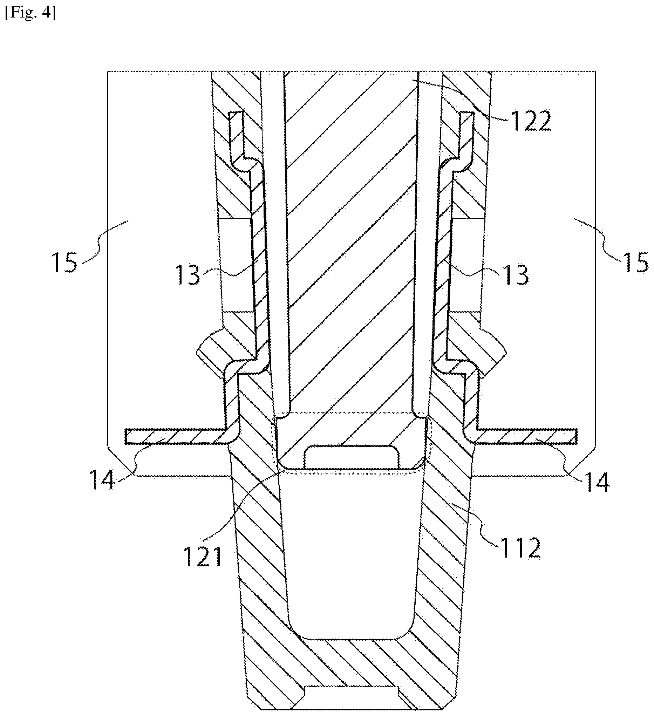

Also, a relief recess for preventing sink marks may be formed at the bottom of the sealing portion 121, as shown in Fig. 4.

In the present invention the sealing portion 121 separates the sealed portion of the sample holding portion 112 and the later described electrodes 13 from each other. With this arrangement, a reagent sealed in part of the sample holding portion 112 can be prevented from scattering onto the inner sidewall of the container 11 and the electrodes 13, in a case where the electrical measurement cartridge 1 having the reagent sealed in part of the sample holding portion 112 is transported or stored, for example. In this manner, an effective amount of the reagent can be maintained for the sample at the time of electrical characteristics measurement, and a measurement error or the like due to the reagent remaining on the electrodes 13 can be reduced.

### (2-2) Shaft portion 122

The shaft portion 122 is a portion extending from the sealing portion 121.

The cross-sectional area of the shaft portion 122 is preferably smaller than the cross-sectional area of the sealing portion 121. In a conventional cartridge, when a user reattaches the lid unit to the container after using the cartridge, the sample after measurement abruptly flows in reverse and is scattered about in some cases. This is because the cross-sectional area of the shaft portion 122 is substantially the same as the cross-sectional area of the sealing portion 121, and there is only a narrow space between the shaft portion 122 and the container 11, increasing the speed of flow of the measured sample passing through the space. To counter this in an embodiment of the present invention, the cross-sectional area of the shaft portion 122 is made smaller than the cross-sectional area of the sealing portion 121, so that the speed of flow of the sample passing through the space between the shaft portion 122 and the container 11 can be reduced, and the risk of back-flow and scattering of the sample after measurement can be lowered.

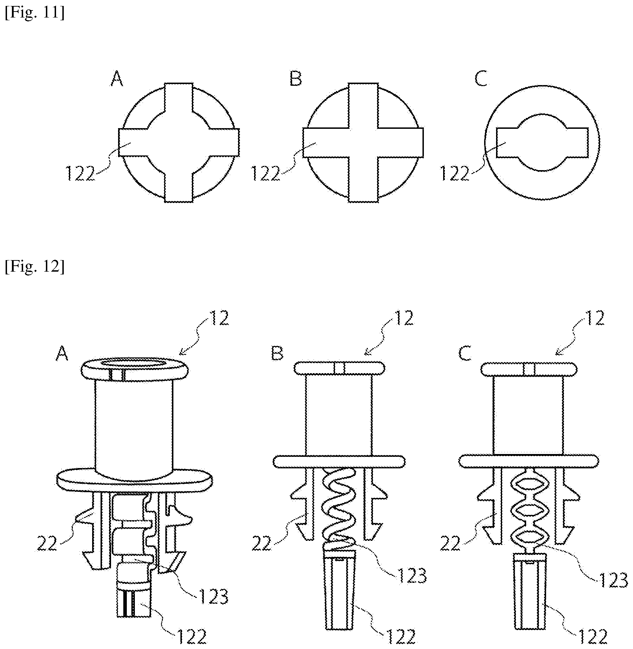

The shaft portion 122 is not limited to any particular cross-sectional shape, and may have, for example, a cross-like shape shown in B of Fig. 11, a bar-like shape, a cross-like or bar-like shape having its center portion formed in a circular shape as shown in Fig. 8, A of Fig. 11, and C of Fig. 11 (a shape formed by reinforcing a cylindrical stick with a cross or a bar), or the like.

### (2-3) Spring portion 123

The electrical measurement cartridge 1 according to an embodiment of the present invention may further include the spring portion 123, as in the embodiment shown in Fig. 8. The spring portion 123 is a portion provided at the top edge on the different side from the side of the shaft portion 122 and the sealing portion 121. As the electrical measurement cartridge 1 according to an embodiment of the present technology includes the spring portion 123, the later described stress generation mechanism can be formed.

The spring portion 123 is not limited to any particular shape, and may have, for example, a wave-like shape shown in Fig. 8 and A of Fig. 12, a coil-like shape shown in B of Fig. 12, a pantograph-like shape shown in C of Fig. 12, or the like.

Further, in a case where the spring portion 123 has a wave-like shape, the direction of formation of waves is not limited to any particular direction. For example, the waves may be formed in the same plane as the later described claws 22 as shown in Fig. 8, or the waves may be formed in a different plane from the claws 22 (for example, in a plane tilted 90 degrees to the same plane as the claws 22) as shown in A of Fig. 12. However, the waves are preferably formed in the same plane as the claws 22 as shown in Fig. 8, so that the lid unit 12 is easily engaged with the container 11.

Further, the spring portion 123 is preferably designed to have such a stretching force that the later described securing mechanism 2 is not easily unlocked.

### (3) Electrodes 13

The electrodes 13 are portions that are secured to the sample holding portion 112 in advance. To electrically measure the state of a sample, the electrodes 13 are brought into contact with the sample at the time of electrical measurement, and applies a necessary voltage to the sample.

The electrodes 13 are preferably formed with at least a pair of electrodes 13 as shown in Fig. 4. However, the electrodes 13 may be formed with more than one pair of electrodes 13, depending on the type of the sample, the measurement method, and the electrical measurement device being used. For example, in a case where the permittivity and the impedance of a sample are measured, more than one pair of electrodes 13 may be provided for the sample holding portion 112. In such a case, to measure the electrical characteristics of a sample, the electrodes 13 are preferably arranged parallel to one another. However, to achieve a high mold releasability in the case of insert molding or the like, the electrodes 13 may be arranged with a tilt of several degrees to one another, for example.

The electrodes 13 are not limited to any particular arrangement or form as long as being capable of applying a necessary voltage to a sample, and can be freely designed in accordance with the form of the container 11, the measurement method, the electrical measurement device being used, and the like. The electrodes 13 are preferably in planar contact with a sample, particularly to achieve a higher measurement efficiency. If there is a step on the inner sidewall of the sample holding portion 112, air bubbles might stay at the step portion, or unevenness might appear in the reagent concentration at the step portion. This adversely affects measurement values. Therefore, the connecting portions between the sample holding portion 112 and the electrodes 13 are smoothed, so that the adverse influence of air bubbles or uneven sample concentration is eliminated. Thus, the measurement accuracy in electrical measurement can be increased.

The electrodes 13 are brought into planar contact with a sample by a method that is not limited to any particular one. For example, the electrodes 13 may be designed to have a great width so that the electrodes 13 are brought into planar contact with a sample.

The electrodes 13 are secured to the sample holding portion 112 by a method that is not limited to any particular one. However, it is preferable to integrally mold the sample holding portion 112 and the electrodes 13, with part of the electrodes 13 being buried in the sample holding portion 112. The reasons for this are described below in detail.

In a case where the electrodes 13 are secured to the sample holding portion 112 with an adhesive agent, for example, the properties of the sample might be adversely affected, depending on the type of the adhesive agent being used. Particularly, in a case where blood is selected as a sample, the blood-coagulating activity is enhanced depending on the type of the adhesive agent being used, and the measurement to be conducted might be adversely affected. However, it is possible to eliminate adverse influence of a securing material such as an adhesive agent on the sample, by adopting a method of integrally molding the sample holding portion 112 and the electrodes 13, or a method of securing the electrodes 13 to the sample holding portion 112 without any securing material such as an adhesive agent.

In addition, even in a case where a securing material that hardly affects a sample is used, the bonding step using the securing material is added to the process of manufacturing a cartridge for housing a sample, and therefore, productivity becomes lower. Where a method of integrally molding the sample holding portion 112 and the electrodes 13 is adopted, on the other hand, there is no need to carry out the bonding step in addition to the step of molding the sample holding portion 112. Accordingly, the manufacturing of the electrical measurement cartridge 1 according to an embodiment of the present invention becomes easier, and cartridges 1 can be mass-produced at low costs.

The method of integrally molding the sample holding portion 112 and the electrodes 13 is not limited to any particular method, and any appropriate method can be adopted. For example, in a case where the sample holding portion 112 is formed with a resin, the electrodes 13 are arranged at predetermined positions when the resin is solidified from a melted state, so that the sample holding portion 112 and the electrodes 13 can be integrally molded. More specifically, the electrodes 13 are inserted into a metal mold, and a resin is injected into the space surrounding the electrodes 13, so that the electrodes 13 and the resin are integrated. This is a method of integrally molding the sample holding portion 112 and the electrodes 13 by "insert molding".

Also, when the sample holding portion 112 is molded, the electrodes 13 are secured to the sample holding portion 112 at the same time. In this manner, the process of manufacturing the electrical measurement cartridge 1 can be simplified. Accordingly, electrical measurement cartridges 1 according to an embodiment of the present invention can be mass-produced at low costs.

Further, by taking advantage of this feature, each electrical measurement cartridge 1 may be disposed of after being used for one sample. With this configuration, the trouble of washing a cartridge can be avoided, and the measurement process can be simplified. A measurement error or the like due to another sample remaining in the cartridge can also be prevented, and a higher measurement accuracy can be achieved in electrical measurement.

Further, although very rare, leakage of a sample or a reagent from a boundary portion between the sample holding portion 112 and the electrodes 13 might occur due to a distortion difference between the resin and an electroconductive material under certain storage conditions or measurement conditions such as temperature. Therefore, part of the portion of each electrode 13 secured to the sample holding portion 112 is bent, and a case with the bent portion is compared with a case without such a bent portion. The comparison results show that, with the bent portion, leakage of a sample from a boundary between the sample holding portion 112 and the electrodes 13 can be more surely prevented. Also, the adhesion of the electrodes 13 to the sample holding portion 112 becomes higher, and thus, a strong electrical measurement cartridge 1 can be formed.

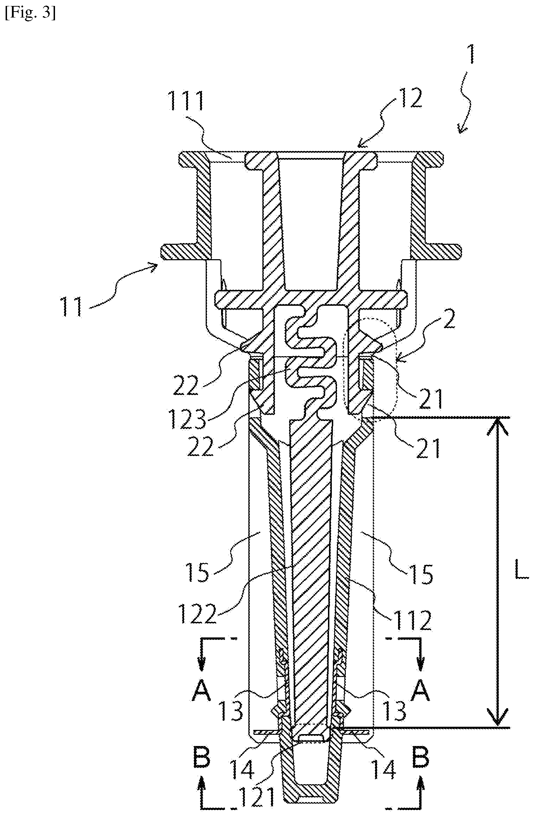

Further, any resin may not be provided at the portions of the electrodes 13 outside the cartridge, as shown in the embodiment shown in Fig. 3. As such arrangement is adopted, the electrodes 13 can be secured to the sample holding portion 112 by vacuum suction when the sample holding portion 112 is molded, for example. Furthermore, as the electrodes 13 are held by the sample holding portion 112 at the time of molding of the sample holding portion 112, deformation of the electrodes 13 can also be prevented.

It should be noted that, the method of securing the electrodes 13 from outside the cartridge is not limited to the vacuum suction, and any method may be adopted, as long as the electrodes 13 can be appropriately secured from outside the cartridge.

### (4) Securing mechanism 2

A securing mechanism 2 is a mechanism that secures the container 11 and the lid unit 12 when the sealing portion 121 seals at least part of the sample holding portion 112. With the securing mechanism 2, detachment of the lid unit 12 from the container 11 can be prevented, and the sealed state of part of the sample holding portion 112 with the sealing portion 121 can be stably maintained.

Although the securing mechanism 2 is not limited to any particular structure, the securing mechanism 2 is formed with recess portions 21 formed in the container 11, and claws 22 that are formed in part of the lid unit 12 and are engaged with the recess portions 21, as in the embodiment shown in Fig. 1. The later described guiding portions 16 may also function as the recess portions 21.

The securing mechanism 2 may be designed to be incapable of re-engagement after release. Since the securing mechanism 2 is incapable of re-engagement after release, it may be impossible for the electrical measurement cartridge 1 that is once opened to re-engage the lid unit 12 with the container 11. Thus, the history of the opening of the electrical measurement cartridge 1 can be checked. Accordingly, in a case where electrical measurement cartridges 1 in which reagents are sealed are put on the market, quality can be guaranteed. It should be noted that this design does not hinder a removed lid unit 12 from being reattached to the container 11 after an end of electrical measurement.

In the description below, the respective portions in a case where the securing mechanism 2 is formed with the recess portions 21 and the claws 22 are explained in detail.

### (4-1) Recess portions 21

The recess portions 21 are portions that are formed in the container 11 and are engaged with the claws 22. The recess portions 21 are not limited to any particular form, and may be holes, grooves, or the like formed in part of the container 11, for example. More specifically, the recess portions 21 may be holes or the like that are designed to penetrate from the inner sidewall to the outer sidewall of the sample holding portion 112, for example.

### (4-2) Claws 22

The claws 22 are portions that are formed in part of the lid unit 12 and are engaged with the recess portions 21. The claws 22 are not limited to any particular form, and may be protrusions or the like formed on both sides of the spring portion 123, for example, as shown in Fig. 8 and others.

The claws 22 may also be designed to be flexible. Specifically, for example, the claws 22 may be formed with a flexible material, such as a resin. Being flexible, the claws 22 can be easily engaged with the recess portions 21, and thus, user-friendliness is increased. Further, the claws 22 and the other portions formed on the lid unit 12 may be formed with one resin. Thus, the manufacturing process can be simplified, and a higher economic efficiency can be achieved.

The claws 22 may also be designed to be deformed or cut off so that it may be impossible for the claws 22 to be re-engaged when the securing mechanism 2 is unlocked. A mechanism of deforming or cutting off the claws 22 is not limited to any particular mechanism. For example, the claws 22 may be melted and then deformed by a chemical method using heat or the like, or the claws 22 may be cut off by a physical method, for example.

Like the container 11 and the lid unit 12, the recess portions 21 and the claws 22 may also be formed with a resin. Further, the recess portions 21, the claws 22, the other portions formed on the container 11, and the other portions formed on the lid unit 12 may be formed with one resin. Thus, the manufacturing process can be simplified, and a higher economic efficiency can be achieved. It should be noted that the types of resins and the preferred resins and the like are similar to those described above, and therefore, explanation of them is not repeated herein.

It should be noted that, although an electrode 13, a recess portion 21, and claws 22 are located on the same side surface of the container 11 in the embodiment shown in Fig. 1, an electrode 13, a recess portion 21, and claws 22 may be located on different side surfaces of the sample holding portion 112. As an electrode 13, a recess portion 21, and claws 22 are located on different side surfaces of the container 11, a user can be prevented from inadvertently bringing the electrode 13 into the unsealing mechanism when inserting the electrical measurement cartridge 1 into the cartridge insertion unit 3 of the later described electrical measurement device 10.

### (5) Stress generation mechanism

The stress generation mechanism is a mechanism that generates stress in at least a portion of the lid unit 12 when the container 11 and the lid unit 12 are secured by the securing mechanism 2. The stress is generated in the direction in which the lid unit 12 extends. With the stress generation mechanism, a pressing force to cause the sealing portion 121 to adhere tightly to the inner wall of the container 11 is obtained, and excellent sealing characteristics can be achieved. Also, by virtue of the stress generation mechanism, excellent sealing characteristics can be achieved even if the lid unit 12 is formed with a resin, and the sealing portion 121 is not formed with an elastic packing.

Also, as the stress generation mechanism is provided, wobbling of the lid unit 12 when the lid unit 12 is engaged with the container 11 can be prevented. Accordingly, in a case where electrical measurement cartridges 1 in which reagents are sealed are put on the market, for example, the risk of reagent leakage from the sample holding portion 112 can be lowered. Further, in a case where the securing mechanism 2 is formed with recess portions 21 and claws 22, it is possible to cause the lid unit 12 to pop up to a position where the lid unit 12 can be easily removed, simply by disengaging the claws 22 from the recess portions 21.

The stress generation mechanism is not limited to any particular structure, but is preferably a mechanism formed with the above described spring portion 123. With this arrangement, a pressing force generated from the stretching force of the spring portion 123 can be obtained without fail.

Further, the stress generation mechanism may be a mechanism that is formed in a case where the length of the above described shaft portion 122 is longer than the length L (see Fig. 3) from the upper edge of the sealing portion 121 to the lower edge of each recess portion 21 when the lid unit 12 is attached to the container 11. With this arrangement, the repulsive force generated due to the extra length absorbed when the lid unit 12 is engaged with the container 11 can be used as a pressing force.

It should be noted that, in the formation of the electrical measurement cartridge 1 according to an embodiment of the present invention, it is of course possible to combine and use two or more stress generation mechanisms.

### (6) Connecting portions 14

The electrical measurement cartridge 1 according to an embodiment of the present invention may further include the connecting portions 14, as in the embodiment shown in Fig. 1. The connecting portions 14 are portions that electrically connect the electrodes 13 to an external circuit. Therefore, the connecting portions 14 are partially connected to the electrodes 13 as in the embodiment shown in Fig. 1.

The connecting portions 14 are not limited to any particular arrangement or form as long as being capable of electrically connecting to an external circuit, and can be freely designed in accordance with the form of the container 11, the measurement method, the electrical measurement device being used, and the like.

The electrodes 13 and the connecting portions 14 are made of an electroconductive material. Electroconductive materials that can be used as the electrodes 13 and the connecting portions 14 are not limited to any particular types, and one or two or more types of materials that can be used for measuring the electrical characteristics of a sample can be freely selected. Examples of such materials include titanium, aluminum, stainless steel, platinum, gold, copper, and black lead.

The electrodes 13 and the connecting portions 14 are preferably formed with a titanium-containing electroconductive material. Titanium has low coagulation activity with respect to blood, and accordingly, can be appropriately used for measurement in a case where a blood sample is selected as a sample.

### (7) Protecting portions 15

The electrical measurement cartridge 1 according to an embodiment of the present invention may further include the protecting portions 15, as in the embodiment shown in Fig. 1. The protecting portions 15 are portions that protect the connecting portions 14. As the electrical measurement cartridge 1 according to an embodiment of the present invention includes the protecting portions 15, it is possible to lower the risk of contact of a user or a manufacturing worker or the like with the connecting portions 14. Accordingly, inadvertent touching of the connecting portions 14 with a finger of a user or a manufacturing worker or the like is reduced, and the possibilities of wounds such as cuts become lower. Thus, safety of the product is increased.

With the protecting portions 15, it is also possible to avoid the risk of deforming or damaging the connecting portions 14 and the risk of the connecting portions 14 ripping the package of the product, at the time of transportation or the like. Further, by virtue of the protecting portions 15, the possibilities of adhesion of an unexpected substance such as dust in the air to the connecting portions 14 are lowered, and the measurement accuracy in electrical measurement can be increased.

The protecting portions 15 are not limited to any particular arrangement or form as long as being capable of protecting the connecting portions 14, and can be freely designed in accordance with the form of the container 11, the measurement method, the electrical measurement device being used, and the like. However, the connecting portions 14 are portions that electrically connect the electrodes 13 to an external circuit, as described above. Therefore, it is difficult to adopt a form in which the connecting portions 14 is completely covered with the protecting portions 15 in the present technology. To counter this, there is a need to provide an opening portion in at least part of each protecting portion 15 so as not to hinder contact of the connecting portions 14 with an external circuit.

Although materials that can be used as the protecting portions 15 are not limited to any particular materials, a resin may be used to form the protecting portions 15. Further, the protecting portions 15 and the other portions formed on the container 11 may be formed with one resin. Thus, the manufacturing process can be simplified, and a higher economic efficiency can be achieved. It should be noted that the types of resins and the preferred resins and the like are similar to those described above, and therefore, explanation of them is not repeated herein.

It should be noted that, although the right and left protecting portions 15 have the same size in the embodiment shown in Fig. 1, the right and left protecting portions 15 may have different sizes from each other. As the right and left protecting portions 15 have different sizes from each other, the position of a bar-code or the like can be marked in a case where the electrical measurement cartridge 1 is managed with the bar-code, for example. Thus, user-friendliness is increased.

### (8) Guiding portions 16

The electrical measurement cartridge 1 according to an embodiment of the present invention may further include the guiding portions 16, as in the embodiment shown in Fig. 1. The guiding portions 16 are portions that guide the electrical measurement cartridge 1 to be inserted into an electrical measurement device. As the electrical measurement cartridge 1 includes the guiding portions 16, the electrical measurement cartridge 1 is smoothly inserted into an electrical measurement device. Also, the position of a bar-code or the like can be marked in a case where the electrical measurement cartridge 1 is managed with a bar-code, for example. Thus, user-friendliness can be increased.

Further, in a case where the later described positioning mechanism is formed with the guiding portions 16, the position of the electrical measurement cartridge 1 in an electrical measurement device can be accurately determined, and measurement errors due to shifting of the electrical measurement cartridge 1 can be reduced.

The guiding portions 16 can also function as the protecting portions 15. Because of this, the protecting portions 15 and the guiding portions 16 can be simultaneously formed, and there is no need to carry out any additional manufacturing step. Accordingly, the manufacturing of the electrical measurement cartridge 1 becomes easier, and electrical measurement cartridges 1 according to an embodiment of the present invention can be mass-produced at low costs. Furthermore, the structure of the electrical measurement device can also be simplified. Thus, the electrical measurement device can be made smaller in size and less expensive.

### (9) Sample

The current sample to be measured is not limited to any particular sample, and may be freely selected. For example, the current sample to be measured may be a biological sample. More specifically, for example, a biological sample may be whole blood, blood plasma, or a blood sample containing a blood component such as a diluted fluid of whole blood or blood plasma and/or a pharmaceutical additive or the like. In addition, in a case where a blood sample is selected as a sample, the electrical measurement cartridge 1 according to an embodiment of the present invention can also be used for measuring a blood coagulation state.

### (10) Reagent

In a case where the electrical measurement cartridge 1 according to an embodiment of the present invention can seal a reagent in part of the sample holding portion 112, the reagent that can be sealed in part of the sample holding portion 112 is not limited to any particular reagent, but can be freely selected. For example, the reagent may be in a solid state, a liquid state, a cluster state, a powdery state, or the like, but is preferably in a solid state. With this configuration, the risk of leakage of the reagent from the lid unit 12 becomes lower, and user-friendliness in transporting or storing the electrical measurement cartridge 1 according to an embodiment of the present invention becomes higher.

In a case where the electrical measurement cartridge 1 according to an embodiment of the present invention can seal a reagent in part of the sample holding portion 112, the cartridge may be transported or stored, with the reagent being sealed in advance. In this case, a user opens the electrical measurement cartridge 1 immediately before conducting measurement, and can promptly start the measurement simply by introducing the current sample to be measured into the sample holding portion 112. Thus, dust or the like in the air, which will cause a decrease in measurement accuracy, can be prevented from entering the sample holding portion 112, and the measurement accuracy is increased. Further, the number of steps to take before starting measurement is decreased, and thus, user-friendliness is increased.

More specific examples of reagents include an anticoagulant and a coagulation initiator. In addition, some kinds of reagents may be cooled, frozen, or freeze-dried in advance. Further, the electrical measurement cartridge 1 according to an embodiment of the present invention may be temporarily cooled or frozen and be stored, with a reagent being sealed in the electrical measurement cartridge 1.

### 2. Electrical measurement device 10

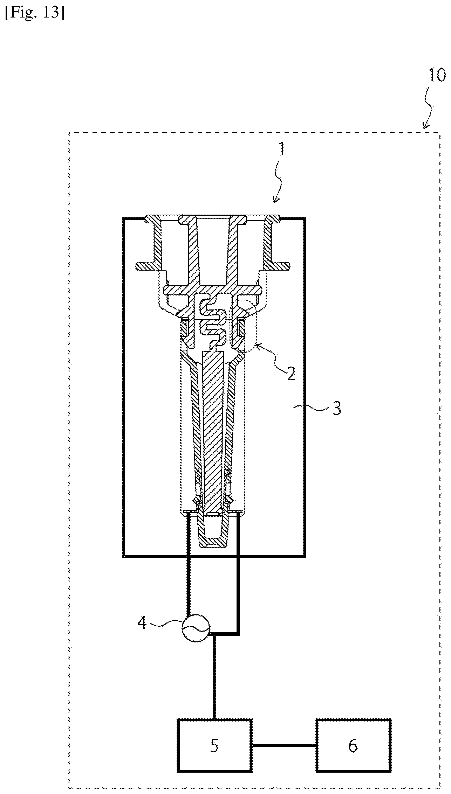

Fig. 13 is a schematic conceptual diagram schematically showing an embodiment of an electrical measurement device 10 according to an embodiment of the present invention. In this embodiment, the electrical measurement cartridge 1 according to the embodiment shown in Fig. 1 is used.

The electrical measurement device 10 according to an embodiment of the present invention includes at least the above described electrical measurement cartridge 1, a cartridge insertion unit 3, an application unit 4, and a measurement unit 5. The electrical measurement device 10 may further include an unsealing mechanism, an analysis unit 6, and the like, as necessary. In the description below, the respective components will be explained in detail. It should be noted that the electrical measurement cartridge 1 is similar to that described above, and therefore, explanation thereof is not repeated herein.

### (1) Cartridge insertion unit 3

The cartridge insertion unit 3 is a component into which the electrical measurement cartridge 1 according to an embodiment of the present invention is inserted. The cartridge insertion unit 3 can be freely designed in accordance with the form of the electrical measurement cartridge 1.

Further, the cartridge insertion unit 3 may have a temperature adjustment mechanism.

The temperature adjustment mechanism is a mechanism that maintains the sample held in the sample holding portion 112 at constant temperature. As the electrical measurement device 10 has the temperature adjustment mechanism, the temperature of a sample becomes constant, and measurement errors due to changes in the temperature of a sample can be reduced.

The temperature adjustment mechanism is not limited to any particular structure, and may be formed with a material that can keep the temperature of the cartridge insertion unit 3, for example.

### (2) Application unit 4

The application unit 4 is a component that applies a voltage to the connecting portions 14 of the electrical measurement cartridge 1 according to an embodiment of the present invention. Specifically, the application unit 4 applies a voltage to the connecting portions 14 of the electrical measurement cartridge 1 at a starting time that is a time when a measurement start instruction is received or a time when power is applied to the electrical measurement device 10. In this case, the application unit 4 applies an AC voltage at a predetermined frequency to the connecting portions 14 at predetermined measurement intervals. It should be noted that, depending on the electrical characteristics to be measured, the voltage to be applied by the application unit 4 may be a DC voltage.

### (3) Measurement unit 5

The measurement unit 5 is a component that measures the electrical characteristics of a sample. Specifically, the measurement unit 5 measures electrical characteristics such as complex permittivity (hereinafter also referred to as "permittivity") and its frequency dispersion, at a starting time that is a time when a measurement start instruction is received or a time when power is applied to the electrical measurement device 10. In a case where permittivity is measured, for example, the measurement unit 5 measures the electric current or the impedance between the electrodes 13 of the electrical measurement cartridge 1 at predetermined intervals, and calculates permittivity from the measurement values. In this permittivity calculation, a known function or relational expression indicating a relationship between electric current or impedance and permittivity can be used.

### (4) Unsealing mechanism

The electrical measurement device 10 according to an embodiment of the present invention may have an unsealing mechanism, for example, in a case where the electrical measurement cartridge 1 can seal a reagent in part of the sample holding portion 112. The unsealing mechanism is a mechanism that cancels a sealed state of at least part of the sample holding portion 112. As the electrical measurement device 10 has the unsealing mechanism, it is possible to lower the risk of application of unnecessary shock to the electrical measurement cartridge 1, and the risk of application of an unnecessary external force to the container 11 or the lid unit 12. Further, as a sealed state of at least part of the sample holding portion 112 can be smoothly canceled by the unsealing mechanism, it is possible to prevent a reagent sealed beforehand in the sealed portion of the sample holding portion 112 from scattering onto the wall surface of the cartridge, and thus, user-friendliness and measurement accuracy are increased.

The unsealing mechanism is not limited to any particular structure, and may cancel the engagement between the container 11 and the lid unit 12 secured by the securing mechanism 2, by a chemical method using heat or the like, or by a physical method or the like, for example. More specifically, when the electrical measurement cartridge 1 is inserted into the cartridge insertion unit 3, an unlocking pin may be provided on the side of the electrical measurement device 10, for example.

### (5) Analysis unit 6

The electrical measurement device 10 according to an embodiment of the present invention may further include the analysis unit 6. The analysis unit 6 is a component that receives data of the electrical characteristics of a sample obtained from the measurement unit 5, and determines the physical properties of the sample.

Specifically, electrical characteristics data of a sample obtained from the measurement unit 5 is supplied to the analysis unit 6 at predetermined measurement intervals, and the analysis unit 6 starts determining the physical properties of the sample, in accordance with the electrical characteristics data supplied from the measurement unit 5. The analysis unit 6 also sends a notification of a result of the determination on the physical properties of the sample and/or permittivity data. This notification can be sent in the form of a graph that is displayed on a monitor or is printed on a predetermined medium, for example.

### (6) Others

In a case where the electrical measurement cartridge 1 according to an embodiment of the present invention includes the guiding portions 16, the electrical measurement device 10 may further include guided portions. The guided portions are portions with which the guiding portions 16 are to be engaged. As the electrical measurement device 10 includes the guided portions, the electrical measurement cartridge 1 can be smoothly inserted into the electrical measurement device 10, and thus, user-friendliness is increased. Further, in a case where the later described positioning mechanism is formed with the guiding portions 16 and the guided portions, the position of the electrical measurement cartridge 1 in the electrical measurement device 10 can be accurately determined, and measurement errors due to shifting of the electrical measurement cartridge 1 can also be reduced.

The electrical measurement device 10 according to an embodiment of the present invention may further include a positioning mechanism. The positioning mechanism is a mechanism that determines the position of the electrical measurement cartridge 1. As the electrical measurement device 10 includes the positioning mechanism, the position of the electrical measurement cartridge 1 in the electrical measurement device 10 can be accurately determined, and the positions of contact between the connecting portions 14 and the application unit 4 become accurate. Thus, measurement errors due to shifting of the electrical measurement cartridge 1 can be reduced.

The positioning mechanism is not limited to any particular structure, and may be formed with positioning pins for determining the position of the electrical measurement cartridge 1 at a certain height with respect to the electrical measurement device 10, for example. The positioning mechanism may also be formed with the guiding portions 16 and the guided portions.

### 3. Electrical measurement kit K

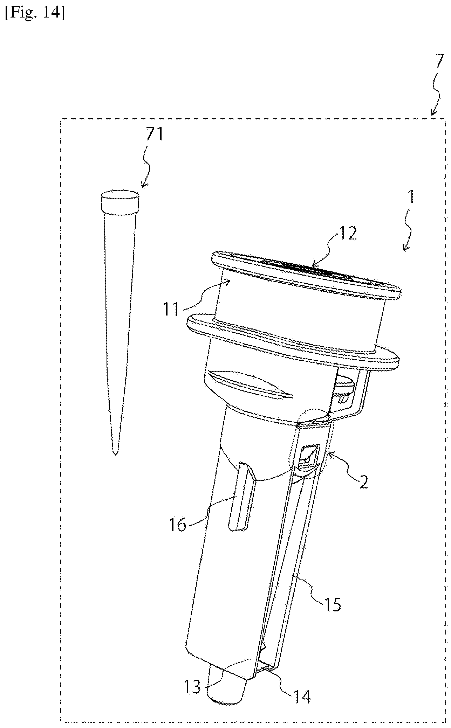

Fig. 14 is a diagram showing an embodiment of an electrical measurement kit K according to an embodiment of the present invention. In this embodiment, the electrical measurement cartridge 1 according to the embodiment shown in Fig. 1 is used.

The electrical measurement kit K according to an embodiment of the present invention includes at least the above described electrical measurement cartridge 1 and a sample introduction member 7. It should be noted that the electrical measurement cartridge 1 is similar to that described above, and therefore, explanation thereof is not repeated herein. In the description below, the sample introduction member 7 will be explained in detail.

### (1) Sample introduction member 7

The sample introduction member 7 is a member for introducing a sample into the sample holding portion 112. For example, the sample introduction member 7 may be a pipette-like chip 71, as in the embodiment shown in Fig. 14. More specifically, the electrical measurement device 10 may be equipped with a suction mechanism (such as a pipetter), and the pipette-like chip 71 is attached to the suction mechanism, so that a sample can be introduced.

The sample introduction member 7 according to an embodiment of the present invention is not limited to the pipette-like chip 71 shown in Fig. 14, but may be freely selected in accordance with the type of the sample, the measurement method, the electrical measurement device being used, and the like, as long as the sample introduction member 7 forms part or all of the instrument that can introduce a sample into the sample holding portion 112. Other than the pipette-like chip 71, the sample introduction member 7 may be an injection needle, for example.

Like each electrical measurement cartridge 1, the sample introduction member 7 may be disposed of after being used once. As the sample introduction member 7 may be disposed of after being used once, the trouble of washing the instruments used for introducing a sample can be avoided, and user-friendliness and measurement efficiency can be increased. A measurement error or the like due to another sample remaining in an instrument used for introducing a sample can also be prevented, and a higher measurement accuracy can be achieved in electrical measurement.

### 4. Electrical measurement method

The electrical measurement cartridge 1 according to an embodiment of the present invention can be used in a preferred manner in an electrical measurement method for measuring the electrical characteristics of a sample. In the electrical measurement method according to an embodiment of the present invention, electrical characteristics that can be measured are not limited to any particular characteristics, and electrical characteristics can be freely measured in accordance with the type of the sample, the physical properties to be analyzed, and the like. For example, the permittivity and the impedance can be measured.

As the electrical measurement method according to an embodiment of the present invention is used, a blood coagulation state or a blood sedimentation state can be analyzed from measurement values of the permittivity and the impedance in a case where blood is selected as the sample. More specifically, from measurement values of the permittivity and/or the impedance received in an analysis period, parameters indicative of the respective features are extracted, and these parameters are compared with reference values that define references for enhancement of blood coagulation and progress in the blood sedimentation process. In accordance with a result of the comparison, a blood coagulation state and a blood sedimentation state can be analyzed.

It should be understood by those skilled in the art that various modifications, combinations, sub-combinations and alterations may occur depending on design requirements and other factors insofar as they are within the scope of the appended claims.

### Reference Signs List

1 Electrical measurement cartridge
11 Container
111 Opening portion
112 Sample holding portion
12 Lid unit
121 Sealing portion
122 Shaft portion
123 Spring portion
13 Electrode
14 Connecting portion
15 Protecting portion
16 Guiding portion
2 Securing mechanism
21 Recess portion
22 Claw
L Length from the upper edge of the sealing portion 121 to the lower edge of each recess portion 21 when the lid unit 12 is attached to the container 11
10 Electrical measurement device
3 Cartridge insertion unit
4 Application unit
5 Measurement unit
6 Analyzing unit
K Electrical measurement kit
71 Pipette-like chip

## Claims

1. An electrical measurement cartridge (1) comprising:
a container (11) having an opening portion and a sample holding portion (112);
a lid (12) including a sealing portion configured to seal with at least part of the sample holding portion (112) and a shaft portion (122) extending from the sealing portion (121), wherein the container (11) is configured to engage with the lid (12) when the sealing portion (121) seals with the at least part of the sample holding portion (112);
an electrode (13) secured to the sample holding portion (112); and
at least one spring (123) configured to generate a pressing force on at least a portion of the lid (12) when the container (11) and the lid (12) are engaged, the pressing force being generated in a direction in which the lid (12) extends;
**characterized in that** the sealing portion (121) is configured to separate a sealed portion of the sample holding portion from the electrode (13).

2. The electrical measurement cartridge according to claim 1, wherein
the at least one spring is positioned at a top edge of the shaft portion on a side different from a side of the shaft portion having the sealing portion.

3. The electrical measurement cartridge according to claim 2, wherein the at least one spring includes at least one of a wave-like shape, a coil-like shape, and a pantograph-like shape.

4. The electrical measurement cartridge according to claim 1, wherein the container further includes a recess portion and the lid further includes a claw configured to engage with the recess portion.

5. The electrical measurement cartridge according to claim 4, wherein the pressing force is generated by the shaft portion having a dimension greater than a length from an upper edge of the sealing portion to a lower edge of the recess portion.

6. The electrical measurement cartridge according to claim 1, wherein
the container further has a sloped inner sidewall configured to contact the sealing portion, the slope of the inner sidewall increasing from a bottom of the container towards the opening portion, and
wherein the sealing portion further has a sloped outer sidewall configured to contact the container, the slope of the outer sidewall increasing from a bottom of the sealing portion toward a top portion of the sealing portion, and
the slope of the outer sidewall of the sealing portion is less than the slope of the inner sidewall of the container.

7. The electrical measurement cartridge according to claim 1, wherein the sealing portion and at least one other portion of the lid are made of the same material.

8. The electrical measurement cartridge according to claim 1, wherein the container and/or the lid is formed with a resin.

9. The electrical measurement cartridge according to claim 8, wherein the resin is at least one resin selected from the group consisting of polypropylene, polystyrene, acrylic, and polysulfone.

10. The electrical measurement cartridge according to claim 1, wherein a cross-sectional area of the shaft portion is smaller than a cross-sectional area of the sealing portion.

11. The electrical measurement cartridge according to claim 10, wherein a cross-sectional shape of the shaft portion is one of a cross-like shape, a bar-like shape, and a cross-like shape or a bar-like shape having a center portion formed in a circular shape.

12. The electrical measurement cartridge according to claim 1, wherein a reagent is sealed in a sealed portion of the sample holding portion.

13. An electrical measurement device (10) comprising:
an electrical measurement cartridge(11) according to any of Claims 1 to 12.

14. An electrical measurement kit (K) comprising:
an electrical measurement cartridge (11) according to any of Claims 1 to 12.

15. An electrical measurement method comprising:
measuring electrical characteristics of a sample, using an electrical measurement cartridge (11) according to any of Claims 1 to 12.

## Patentansprüche

1. Elektrische Messkartusche (1), umfassend:
einen Behälter (11), der einen Öffnungsabschnitt und einen Probenaufnahmeabschnitt (112) aufweist;
einen Deckel (12), der einen Versiegelungsabschnitt, der dazu ausgelegt ist, wenigstens einen Teil des Probenaufnahmeabschnitts (112) zu versiegeln, und einen Schaftabschnitt (122) umfasst, der sich von dem Versiegelungsabschnitt (121) erstreckt, wobei der Behälter (11) dazu ausgelegt ist, mit dem Deckel (12) einzugreifen, wenn der Versiegelungsabschnitt (121) den wenigstens einen Teil des Probenaufnahmeabschnitts (112) versiegelt;
eine Elektrode (13), die an dem Probenaufnahmeabschnitt (112) befestigt ist; und
wenigstens eine Feder (123), die dazu ausgelegt ist, eine Presskraft auf wenigstens einen Abschnitt des Deckels (12) zu erzeugen, wenn sich der Behälter (11) und der Deckel (12) in Eingriff befinden, wobei die Presskraft in einer Richtung erzeugt wird, in der sich der Deckel (12) erstreckt;
**dadurch gekennzeichnet, dass** der Versiegelungsabschnitt (121) dazu ausgelegt ist, einen versiegelten Abschnitt des Probenaufnahmeabschnitts von der Elektrode (13) zu trennen.

2. Elektrische Messkartusche nach Anspruch 1, wobei die wenigstens eine Feder an einem oberen Rand des Schaftabschnitts auf einer Seite positioniert ist, die sich von einer Seite des Schaftabschnitts, die den Versiegelungsabschnitt aufweist, unterscheidet.

3. Elektrische Messkartusche nach Anspruch 2, wobei die wenigstens eine Feder eine wellenähnliche Form und/oder eine spulenähnliche Form und/oder eine storchschnabelähnliche Form umfasst.

4. Elektrische Messkartusche nach Anspruch 1, wobei der Behälter ferner einen Vertiefungsabschnitt umfasst und der Deckel ferner eine Klaue umfasst, die dazu ausgelegt ist, mit dem Vertiefungsabschnitt einzugreifen.

5. Elektrische Messkartusche nach Anspruch 4, wobei die Presskraft dadurch erzeugt wird, dass der Schaftabschnitt eine Abmessung aufweist, die größer als eine Länge von einem oberen Rand des Versiegelungsabschnitts zu einem unteren Rand des Vertiefungsabschnitts ist.

6. Elektrische Messkartusche nach Anspruch 1, wobei
der Behälter ferner eine geneigte innere Seitenwand aufweist, die dazu ausgelegt ist, den Versiegelungsabschnitt zu kontaktieren, wobei die Neigung der inneren Seitenwand von einem Boden des Behälters zu dem Öffnungsabschnitt zunimmt, und
wobei der Versiegelungsabschnitt ferner eine geneigte äußere Seitenwand aufweist, die dazu ausgelegt ist, den Behälter zu kontaktieren, wobei die Neigung der äußeren Seitenwand von einem Boden des Versiegelungsabschnitts zu einem oberen Abschnitt des Versiegelungsabschnitts zunimmt, und
die Neigung der äußeren Seitenwand des Versiegelungsabschnitts geringer als die Neigung der inneren Seitenwand des Behälters ist.

7. Elektrische Messkartusche nach Anspruch 1, wobei der Versiegelungsabschnitt und wenigstens ein weiterer Abschnitt des Deckels aus dem gleichen Material hergestellt sind.

8. Elektrische Messkartusche nach Anspruch 1, wobei der Behälter und/oder der Deckel mit einem Harz ausgebildet sind bzw. ist.

9. Elektrische Messkartusche nach Anspruch 8, wobei das Harz wenigstens ein Harz ist, das aus der Gruppe ausgewählt ist, die aus Polypropylen, Polystyrol, Acryl und Poylsulfon besteht.

10. Elektrische Messkartusche nach Anspruch 1, wobei eine Querschnittsfläche des Schaftabschnitts kleiner als eine Querschnittsfläche des Versiegelungsabschnitts ist.

11. Elektrische Messkartusche nach Anspruch 10, wobei eine Querschnittsform des Schaftabschnitts eine kreuzähnliche Form und/oder eine balkenähnliche Form und/oder eine kreuzähnliche Form oder eine balkenähnliche Form, die einen in einer kreisrunden Form ausgebildeten mittleren Abschnitt aufweist, ist.

12. Elektrische Messkartusche nach Anspruch 1, wobei ein Reagens in einem versiegelten Abschnitt des Probenaufnahmeabschnitts versiegelt ist.

13. Elektrische Messvorrichtung (10), umfassend:
eine elektrische Messkartusche (11) nach einem der Ansprüche 1 bis 12.

14. Elektrisches Messkit (K), umfassend:
eine elektrische Messkartusche (11) nach einem der Ansprüche 1 bis 12.

15. Elektrisches Messverfahren, umfassend:
Messen von elektrischen Eigenschaften einer Probe unter Verwendung einer elektrischen Messkartusche (11) nach einem der Ansprüche 1 bis 12.

## Revendications

1. Cartouche de mesure électrique (1) comprenant :
un récipient (11) possédant une partie d'ouverture et une partie (112) renfermant un échantillon ;
un couvercle (12) comprenant une partie d'étanchéité conçue pour fermer hermétiquement au moins une partie de la partie (112) renfermant un échantillon et une partie tige (122) s'étendant depuis la partie d'étanchéité (121), dans laquelle le récipient (11) est conçu pour entrer en prise avec le couvercle (12) lorsque la partie d'étanchéité (121) ferme hermétiquement l'au moins une partie de la partie (112) renfermant un échantillon ;
une électrode (13) fixée à la partie (112) renfermant un échantillon ; et
au moins un ressort (123) conçu pour générer une force de pression sur au moins une partie du couvercle (12) lorsque le récipient (11) et le couvercle (12) sont en prise, la force de pression étant générée dans une direction dans laquelle s'étend le couvercle (12) ;
**caractérisée en ce que** la partie d'étanchéité (121) est conçue pour séparer de l'électrode (13) une partie hermétique de la partie renfermant un échantillon.

2. Cartouche de mesure électrique selon la revendication 1, dans laquelle
l'au moins un ressort est positionné au niveau d'un bord supérieur de la partie tige sur un côté différent d'un côté de la partie tige comportant la partie d'étanchéité.

3. Cartouche de mesure électrique selon la revendication 2, dans laquelle l'au moins un ressort comprend une forme de vague et/ou une forme d'enroulement et/ou une forme de pantographe.

4. Cartouche de mesure électrique selon la revendication 1, dans laquelle le récipient comprend en outre une partie évidée et le couvercle comprend en outre une griffe conçue pour venir en prise avec la partie évidée.

5. Cartouche de mesure électrique selon la revendication 4, dans laquelle la force de pression est générée par la partie tige ayant une dimension supérieure à une longueur depuis un bord supérieur de la partie d'étanchéité vers un bord inférieur de la partie évidée.

6. Cartouche de mesure électrique selon la revendication 1, dans laquelle
le récipient comprend en outre une paroi latérale interne inclinée conçue pour entrer en contact avec la partie d'étanchéité, la pente de la paroi latérale interne augmentant depuis un fond du récipient vers la partie d'ouverture, et
dans laquelle la partie d'étanchéité comporte en outre une paroi latérale externe inclinée conçue pour entrer en contact avec le récipient, la pente de la paroi latérale externe augmentant depuis un fond de la partie d'étanchéité vers une partie supérieure de la partie d'étanchéité, et
la pente de la paroi latérale externe de la partie d'étanchéité est inférieure à la pente de la paroi latérale interne du récipient.

7. Cartouche de mesure électrique selon la revendication 1, dans laquelle la partie d'étanchéité et au moins une autre partie du couvercle sont faites du même matériau.

8. Cartouche de mesure électrique selon la revendication 1, dans laquelle le récipient et/ou le couvercle sont formés d'une résine.

9. Cartouche de mesure électrique selon la revendication 8, dans laquelle la résine est au moins une résine sélectionnée dans le groupe consistant en polypropylène, polystyrène, acrylique et polysulfone.

10. Cartouche de mesure électrique selon la revendication 1, dans laquelle une zone de coupe transversale de la partie tige est plus petite qu'une zone de coupe transversale de la partie d'étanchéité.

11. Cartouche de mesure électrique selon la revendication 10, dans laquelle une forme de coupe transversale de la partie tige est une forme de croix et/ou une forme de barre et/ou une forme de croix ou une forme de barre ayant une partie centrale de forme circulaire.

12. Cartouche de mesure électrique selon la revendication 1, dans laquelle un réactif est scellé dans une partie hermétique de la partie renfermant un échantillon.

13. Dispositif de mesure électrique (10) comprenant :
une cartouche de mesure électrique (11) selon l'une quelconque des revendications 1 à 12.

14. Kit de mesure électrique (K) comprenant :
une cartouche de mesure électrique (11) selon l'une quelconque des revendications 1 à 12.

15. Procédé de mesure électrique comprenant :
la mesure de caractéristiques électriques d'un échantillon, au moyen d'une cartouche de mesure électrique (11) selon l'une quelconque des revendications 1 à 12.
